# EUROPEAN PATENT APPLICATION

(11) **EP 2 253 613 A1**
(43) Date of publication of application: **24.11.2010**
(21) Application number: 09717839.6
(22) Date of filing: 03.03.2009
(51) Int. Cl.: C07C 403/24, B01J 31/02

(54) **SULFONE COMPOUND AND CAROTENOID MANUFACTURING METHOD USING SAID COMPOUND**

(30) Priority: 04.03.2008 JP 2008053040; 16.05.2008 JP 2008129199; 22.01.2009 JP 2009012184
(71) Applicant: Sumitomo Chemical Company, Limited, Tokyo 104-8260 (JP)
(72) Inventor: TAKAHASHI, Toshiya, Toyonaka-shi Osaka 560-0021 (JP); OZTURK, Orhan, Toyonaka-shi Osaka 561-0802 (JP)
(74) Representative: Henkel, Feiler & Hänzel
(86) International application number: PCT/JP2009/053933
(87) International publication number: WO 2009/110451

(57) **Abstract**

The present invention relates to a process for producing a sulfone compound of the following formula (3), **characterized in that** an allyl sulfone compound of the formula (1) and an allyl halide compound of the formula (2) are reacted in an organic solvent in the presence of an alkali metal hydroxide and a phase-transfer catalyst: 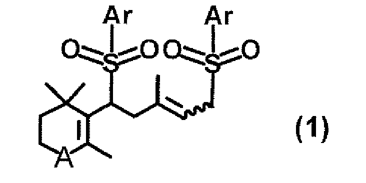 wherein A is CH₂ or C=O; Ar is an aryl group optionally having 1 to 3 substitutents; and the wavy line means that the steric relation to the double bond which the wavy line is bound to is of E-form, Z-form or a mixture of E/Z; 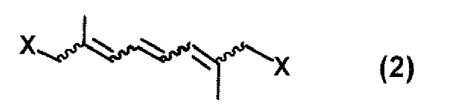 wherein X is a halogen atom; and the wavy line means the same as defined above; and 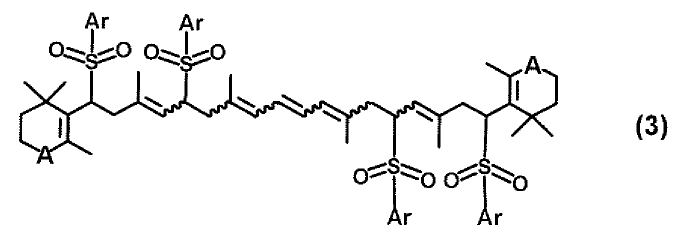 wherein A, Ar and the wavy line mean the same as defined above.

The present invention also relates to a process for producing a carotenoid from the same sulfone compound.

## Description

### TECHNICAL FIELD

The present invention relates to sulfone compound and a process for preparation thereof. The present invention also relates to a process for producing a carotenoid using the same sulfone compound.

### BACKGROUND ART

Carotenoids such as β-carotene, canthaxanthin and astaxanthin conventionally have been used as feed additives, food coloring agents, etc. The existing processes for producing β-carotenes are described in Non-Patent Publication 1: Pure & Appl. Chem., Vol. 63, No. 1, pp.45-58, 1991, in which concretely, a process for producing a C₄₀ β-carotene from two molecules of a C₁₅ Wittig reagent and one molecule of a C₁₀ dialdehyde is described.

### DISCLOSURE OF INVENTION

### PROBLEM TO BE SOLVED BY THE INVENTION

Under such a circumstance, there has been demanded development of intermediates from which carotenoids are easily derived, and processes for producing such intermediates, superior from the viewpoints of costs of starting materials, number of productions steps, purifying step, etc.

### MEANS FOR SOLVING THE PROBLEM

As a result of the present inventors' intensive studies for solving the above-described problem, it is found that a sulfone compound can be produced in one step by a coupling reaction of an ally sulfone compound with an ally halide in the presence of an inexpensive alkali metal hydroxide as a base, under mild conditions, and it is also found that a carotenoid can be readily produced from this solufone compound. The present invention is accomplished based such findings.

Objects and preferable embodiments of the present invention will be described below.
[1] A process for producing a sulfone compound of the following formula (3), **characterized in that** an allyl sulfone compound of the formula (1) and an allyl halide compound of the formula (2) are reacted in an organic solvent in the presence of an alkali metal hydroxide and a phase-transfer catalyst: 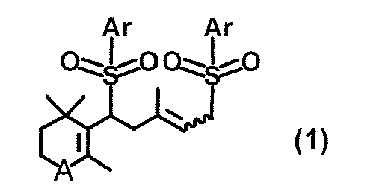 wherein A is CH₂ or C=O; Ar is an aryl group optionally having 1 to 3 substitutents; and the wavy line means that the steric relation to the double bond which the wavy line is bound to is of E-form, Z-form or a mixture of E/Z; 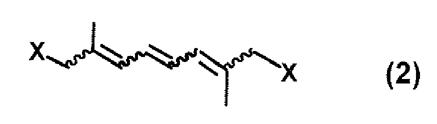 wherein X is a halogen atom; and the wavy line means the same as defined above; and 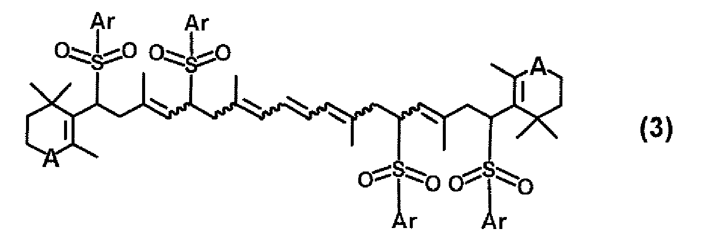 wherein A, Ar and the wavy line mean the same as defined above.
[2] A process for producing a carotenoid, **characterized in that** an allyl sulfone compound of the formula (1) and an allyl halide compound of the formula (2) are reacted in an organic solvent in the presence of an alkali metal hydroxide and a phase-transfer catalyst, and in that an alcohol is added to the resulting reaction mixture: 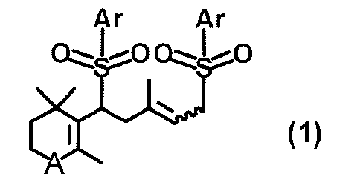 wherein A is CH₂ or C=O; Ar is an aryl group optionally having 1 to 3 substitutents; and the wavy line means that the steric relation to the double bond which the wavy line is bound to is of E-form, Z-form or a mixture of E/Z; and 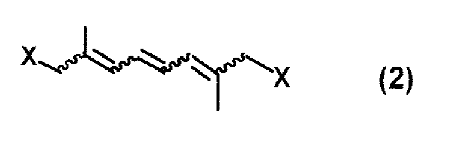 wherein X is a halogen atom; and the wavy line means the same as defined above.
[3] The process defined in the item [1] or [2], wherein in the compound of the formula (1) Ar is a 4-methylphenyl group.
[4]The process defined in any one of the items [1] to [3], wherein in the allyl halide compound of the formula (2) X is a bromine atom or a chlorine atom.
[5] The process defined in the item [4], wherein X is a chlorine atom.
[6]The process defined in any one of the items [1] to [5], wherein the alkali metal hydroxide is potassium hydroxide or sodium hydroxide, having a purity of 85% or higher.
[7] The process defined in any of the items [1] to [6], wherein the particle size of the alkali metal hydroxide is 3 mm or less.
[8] The process defined in the item [7], wherein the particle size of the alkali metal hydroxide is 100 µm or less.
[9] The process defined in any one of the items [1] to [8], wherein the amount of the alkali metal hydroxide to be used is from 1 to 30 times larger, in terms of mole, than that of the allyl sulfone compound of the formula (1).
[10] The process defined in any one of the items [1] to [9], wherein the phase-transfer catalyst is a quaternary ammonium salt.
[11] The process defined in any one of the items [1] to [10], wherein the amount of the phase-transfer catalyst to be used is from 0.01 to 0.5 times larger, in terms of mole, than that of the ally sulfone compound of the formula (1).
[12] The process defined in any one of the items [1] to [11], wherein water is added in an amount from 0.05 to 0.5 times larger, in terms of mole, than that of the ally sulfone compound of the formula (1).
[13] The process defined in any one of the items [1] to [12], wherein the organic solvent is an aromatic hydrocarbon- or ether-based solvent.
[14] A process for producing a carotenoid, **characterized in that** a sulfone compound of the formula (3) is reacted in an organic solvent in the presence of an alkali metal hydroxide and a phase-transfer catalyst: 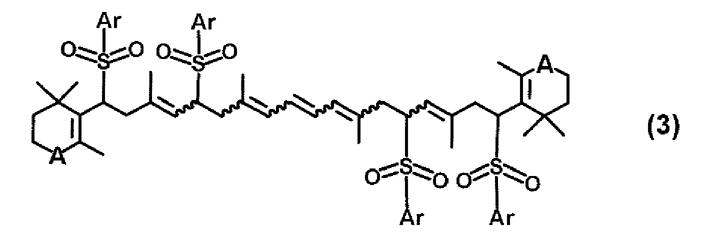 wherein A is CH₂ or C=O; Ar is an aryl group optionally having 1 to 3 substitutents; and the wavy line means that the steric relation to the double bond which the wavy line is bound to is of E-form, Z-form or a mixture of E/Z.
[15] The process defined in the item [14], wherein in the compound of the formula (3) Ar is a 4-methylphenyl group.
[16] The process defined in the item [14] or [15], wherein the alkali metal hydroxide is potassium hydroxide or sodium hydroxide, having a purity of 85% or higher.
[17] The process defined in any one of the items [14] to [16], wherein the particle size of the alkali metal hydroxide is 3 mm or less.
[18] The process defined in the item [17], wherein the particle size of the alkali metal hydroxide is 100 µm or less.
[19] The process defined in any one of the items [14] to [18], wherein the phase-transfer catalyst is a quaternary ammonium salt.
[20] The process defined in any one of the items [14] to [19], wherein a C₁-C₅ lower alcohol is added.
[21] The process defined in any one of the items [14] to [20], wherein the organic solvent is an aromatic hydrocarbon- or ether-based solvent.
[22] A sulfone compound of the formula (3): 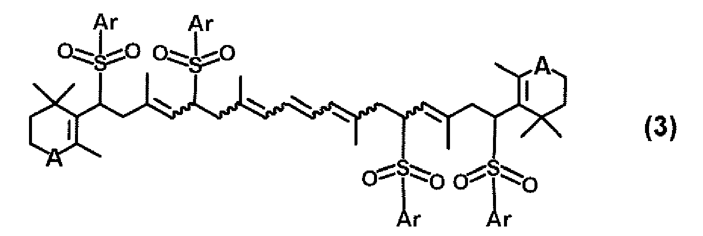 wherein A is CH₂ or C=O; Ar is an aryl group optionally having 1 to 3 substitutents; and the wavy line means that the steric relation to the double bond which the wavy line is bound to is of E-form, Z-form or a mixture of E/Z.
[23] The sulfone compound defined in the time [22], wherein Ar is a 4-methylphenyl group.

### EFFECT OF THE INVENTION

According to the present invention, sulfone compounds useful as intermediates for carotenoids, and carotenoids can be produced by commercially advantageous processes.

### BEST MODES FOR CARRYING OUT THE INVENTION

Hereinafter, the present invention will be described in detail.

In the compounds of the above-described formulas (1) and (3), A is CH₂ or C=O.

In the compounds of the above-described formulas (1) and (3), Ar is an aryl group optionally having 1 to 3 substituents. As the aryl group, there are exemplified a phenyl group, a naphthyl group, etc.; and as the substituents, there are exemplified a C₁-C₅ linear or branched alkyl group, a C₁-C₅ linear or branched alkoxy group, a halogen atom, a nitro group, etc. The aryl group is preferably a phenyl group; and the substitutent is preferably a C₁-C₅ linear or branched alkyl group.

Specific examples of Ar include phenyl, naphthyl, 2-methylphenyl, 3-methylphenyl, 4-methylphenyl, 4-ethylphenyl, 4-propylphenyl, 4-butylphenyl, 4-pentylphenyl, 2-methoxyphenyl, 3-methoxyphenyl, 4-methoxyphenyl, 2-fluorophenyl, 3-fluorophenyl, 4-fluorophenyl, 2-chlorophenyl, 3-chlorophenyl, 4-chlorophenyl, 2-bromophenyl, 3-bromophenyl, 4-bromophenyl, 2-iodephenyl, 3-iodephenyl, 4-iodephenyl, 2-nitorophenyl, 3-nitorophenyl, 4-nitorophenyl, 2,4-dimethylphenyl, 2,4-dimethoxyphenyl, 2,4-dichrolophenyl, 2,4,6-trimethylphenyl, 2,4,6-trichrolophenyl, etc. Preferably, Ar is an unsubstituted phenyl group or a phenyl group substituted by a C₁-C₅ linear or branched alkyl group. More preferably, Ar is a phenyl group which is substituted at its position 4 by a C₁-C₅ linear alkyl group. Particularly, Ar is 4-methylphenyl.

In the allyl halide of the formula (2), X is a halogen atom, specifically a chlorine atom, a bromine atom or an iodine atom, and it is preferably a chlorine atom or a bromine atom, more preferably a chlorine atom, from the viewpoints of production cost, stability and ease of handling.

The sulfone compound of the above-described formula (3) [hereinafter optionally referred to as the sulfone compound (3)] can be obtained by reacting the allyl sulfone compound of the formula (1) [hereinafter optionally referred to as the allyl sulfone compound (1)] and the allyl halide compound of the formula (2) [hereinafter optionally referred to as the allyl halide compound (2)] in an organic solvent in the presence of an alkali metal hydroxide and a phase-transfer catalyst: 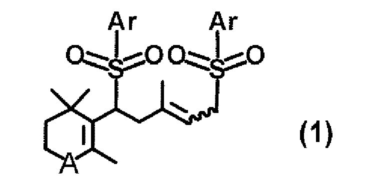 wherein A is CH₂ or C=O; Ar is an aryl group optionally having 1 to 3 substitutents; and the wavy line means that the steric relation to the double bond which the wavy line is bound to is of E-form, Z-form or a mixture of E/Z; and 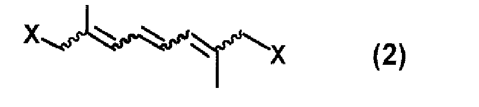 wherein X is a halogen atom; and the wavy line means the same as defined above.

As the alkali metal hydroxide to be used in the above-described reaction, there are exemplified lithium hydroxide, sodium hydroxide, potassium hydroxide, etc., among which potassium hydroxide and sodium hydroxide are preferable.

The amount of the alkali metal hydroxide to be used is usually from 0.5 to 50 times larger, preferably from 1 to 30 times larger, particularly from about 2 to about 20 times larger, in terms of mole, than the amount of the allyl sulfone compound (1). The purity of the alkali metal hydroxide is preferably 85% or higher, particularly 95% or higher.

As the alkali metal hydroxide, a commercially available product in the-form of flakes or pellets may be used as it is. Otherwise, the alkali metal hydroxide may be ground or molten so as to be reduced in particle size. The method of reducing the particle size is not limited: that is, the alkali metal hydroxide may be ground by way of dry grinding or wet grinding in an organic solvent; or otherwise, the alkali metal hydroxide may be molten in an organic solvent for use; or, in some cases, preferably, an anti-agglomeration agent such as polyethylene glycol may be added to the alkali metal hydroxide. While the particle size of the ground or molten alkali metal hydroxide is not limited, it is preferably 3 mm or less, more preferably 2 mm or less. While there is no limit in selection of the lower limit of the particle size, it is usually from about 0.01 to about 100 µm.

The above-described reaction is accelerated by addition of the phase-transfer catalyst. As the phase-transfer catalyst, there are exemplified quaternary ammonium salt, quaternary phosphonium salt and sulfonium salt, etc., among which quaternary ammonium salt is preferable.

Examples of the quaternary ammonium salt include tetramethylammonium chloride, tetraethylammonium chloride, tetrapropylammonium chloride, tetrabutylammonium chloride, tetrapentylammonium chloride, tetrahexylammonium chloride, tetraheptylammonium chloride, tetraoctylammonium chloride, trioctylmethylammonium chloride, tetradecylammonium chloride, tridecylmethylammonium chloride, didecyldimethylammonium chloride, tetradodecylammonium chloride, tridodecylmethylammonium chloride, didodecyldimethylammonium chloride, dodecyltrimethylammonium chloride, dodecyltriethylammonium chloride, tetradecyltrimethylammonium chloride, tetrahexadecylammonium chloride, hexadecyltrimethylammonium chloride, hexadecyldimethylethylammonium chloride, tetraoctadecylammonium chloride, octadecyltrimethylammonium chloride, octadecyltriethylammonium chloride, benzyltrimethylammonium chloride, benzyltriethylammonium chloride, benzyltributhylammonium chloride, 1-methylpyridinium chloride, 1-hexadecylpyridinium chloride, 1,4-dimethylpyridinium chloride and trimethlycyclopropylammonium chloride; or compounds which are bromide salts, iodide salts or hydrogensulfates corresponding to these chloride salts.

Examples of the quaternary phosphonium salt include tributylmethylphosphonium chloride, triethylmethylphosphonium chloride, methyltriphenoxyphosphonium chloride, butyltriphenylphosphonium chloride, tetrabutylphosphonium chloride, benzyltriphenylphosphonium chloride, tetraoctylphosphonium chloride, hexadecyltrimethylphosphonium chloride, hexadecyltributylphosphonium chloride, hexadecyldimethylethylphosphonium chloride and tetraphenylphosphonium chloride; or compounds which are bromide salts or iodide salts corresponding to these chloride salts.

Examples of the sulfonium salt include benzylmethylethylsulfonium chloride, benzyldimethylsulfonium chloride, benzyldiethylsulfonium chloride, dibutylmethylsulfonium chloride, trimethylsulfonium chloride, triethylsulfonium chloride and tributylsulfonium chloride; or compounds which are bromide salts or iodide salts corresponding to these chloride salts.

The amount of such a phase-transfer catalyst to be used is usually from 0.005 to 2 times larger, preferably from about 0.01 to about 0.5 times larger, in terms of mole, than the amount of the allyl sulfone compound (1).

The above-described reaction may be accelerated by addition of water. The amount of water to be added is usually from 0.01 to 1 times larger, preferably from 0.05 to 0.5 times larger, in terms of mole, than the amount of the allyl sulfone compound (1).

The above-described reaction is carried out in an organic solvent. Examples of the organic solvent include hydrocarbon solvents such as n-hexane, cyclohexane, n-pentane, benzene, toluene, xylene, monochlorobenzene and dichlorobenzene; ester solvents such as ethyl acetate; aprotic polar solvents such as acetonitrile, N,N-dimethylformamide, dimethylsulfoxide, hexamethylphosphoric triamide, sulfolane, 1,3-dimethyl-2-imidazolidinone and 1-methyl-2-pyrrolidinone; ether solvents such as diethyl ether, tetrahydrofuran, methyl-t-butyl ether, cyclopentylmethyl ether, 1,4-dioxane, dimethoxyethane, anisole, diglyme, triglyme and tetraglyme; and alcohol solvents such as methanol, ethanol, isopropylalcohol and t-butanol. Among those, the hydrocarbon solvents or the ether solvents are preferable, and aromatic hydrocrabons are particularly preferable. Each of those solvents may be used alone, or two or more selected therefrom may be used as a mixture.

A reaction temperature may be optionally selected within a range of from -78°C to a boiling point of a solvent. Desirably, an optimal reaction temperature should be selected in accordance with the kinds and amounts of material compounds, an alkali metal hydroxide, a phase-transfer catalyst and a solvent. The reaction temperature is preferably from 0 to 70°C, more preferably from 15 to 50°C.

A reaction time may vary depending on the conditions such as the kinds of material compounds, an alkali metal hydroxide and a phase-transfer catalyst, an amount of water added, a solvent and a reaction temperature. The reaction time is usually from about 10 minutes to about 48 hours.

The reaction is carried out preferably under an anoxia condition, desirably under an atmosphere of an inert gas (e.g., nitrogen or argon), using a sufficiently degassed solvent. It is preferable to add a stabilizer before the reaction, and as the stabilizer, there is used an antioxidant such as 3,5-di-t-butyl-4-hydroxytoluene (BHT), 2-t-butyl-4-hydroxyanisole, 3-t-butyl-4-hydroxyanisole, Vitamin E or ethoxyquin, or a mixture thereof.

The sulfone compound (3) and carotenoid are produced through the foregoing reaction. A production ratio between the sulfone compound (3) and carotenoid varies depending on the conditions such as the kinds and amounts of the alkali metal hydroxide and the phase-transfer catalyst, the amount of water added, the kind of the solvent, and the temperature and time for the reaction. Generally, the proportion of the produced sulfone compound (3) is increased, when the amounts of the alkali metal hydroxide and/or the phase-transfer catalyst are smaller and the reaction time is shorter. On the other hand, the proportion of the produced carotenoid is increased, when the amounts of the alkali metal hydroxide and/or the phase-transfer catalyst are larger and the reaction time is longer.

After completion of the reaction, the sulfone compound (3) and the carotenoid may be isolated and purified by an operation for a conventional post-treatment, for example, extraction, washing, crystallization, chromatography or the like.

A carotenoid can be easily derived from a reaction of the sulfone compound (3) with a base. The carotenoid resulting from this reaction is usually obtained as a mixture of geometrical isomers.

It is also possible to produce carotenoid without isolating its intermediate by the following procedure: that is, the allyl sulfone compound (1) is reacted with the allyl halide compound (2) in the organic solvent in the presence of the alkali metal hydroxide and the phase-transfer catalyst; and an alcohol is added to the resulting reaction mixture.

In this case, preferably, the alkali metal hydroxide is added several times in portions, rather than at once when the reaction is started. While timings for adding the alkali metal hydroxide in portions are not limited, preferably, the alkali metal hydroxide is added, when the reaction of the allyl sulfone compound (1) with the allyl halide compound (2) is started, and after the allyl sulfone compound (1) has been substantially dissipated.

Depending on the kind of the reaction solvent, leaving of the sulfonyl group is likely to be efficiently accelerated by addition of a lower alcohol. As the alcohol to be added, C₁-C₅ lower alcohols are preferably used. Specific examples of such alcohols include methanol, ethanol, isopropylalcohol, t-butanol and the like.

An amount of the alcohol to be added is usually from 0.01 to 10 times larger, preferably from 0.1 to 5 times larger, in terms of mole, than the amount of the allyl sulfone compound (1).

Preferably, the alcohol is added at a timing after the allyl sulfone compound (1) as the starting compound has been substantially dissipated.

A solvent, a reagent and conditions for use in production of carotenoid from the isolated sulfone compound (3) may be the same ones as those for use in the method of producing carotenoid from the allyl solufone compound (1) and the allyl halide compound (2) without isolating an intermediate thereof. Specifically, the amount of the alkali metal hydroxide to be used is usually from 0.5 to 50 times larger, preferably from 1 to 30 times larger, more preferably from about 2 to about 20 times larger, in terms of mole, than the amount of the sulfone compound (3); the amount of the phase-transfer catalyst to be used is usually from 0.005 to 2 times larger, preferably from about 0.01 to about 0.5 times larger, in terms of mole, than the amount of the sulfone compound (3); the amount of the alcohol to be added is usually from 0.01 to 10 times larger, preferably from about 0.1 to about 5 times larger, in terms of mole, than the amount of the sulfone compound (3); and the amount of water to be added is usually from 0.01 to 1 times larger, preferably from about 0.05 to about 0.5 times larger, in terms of mole, than the amount of the sulfone compound (3). The reaction temperature is from -78°C to a boiling point of a solvent, preferably from 0 to 70°C, more preferably from 15 to 50°C.

An allyl sulfone compound of the following formula (1-a) [hereinafter optionally referred to as an ally sulfone compound (1-a)] as the starting compound for use in the process of the present invention can be produced by the process disclosed in European Patent Laid-Open Publication No. 1199303. An allyl sulfone compound of the following formula (1-b) [hereinafter optionally referred to as an ally sulfone compound (1-b)] can be produced by subjecting the allyl sulfone compound (1-a) to an oxidation reaction, as described below. Further, the allyl halide compound (2) can be produced, for example, by the following process: 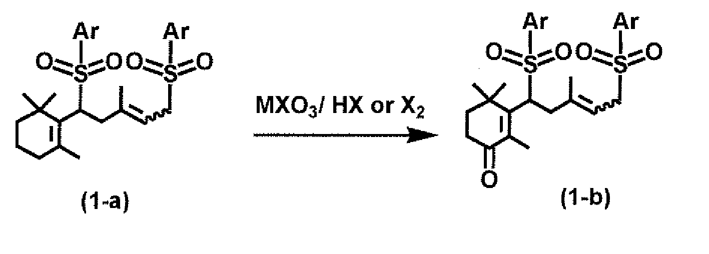 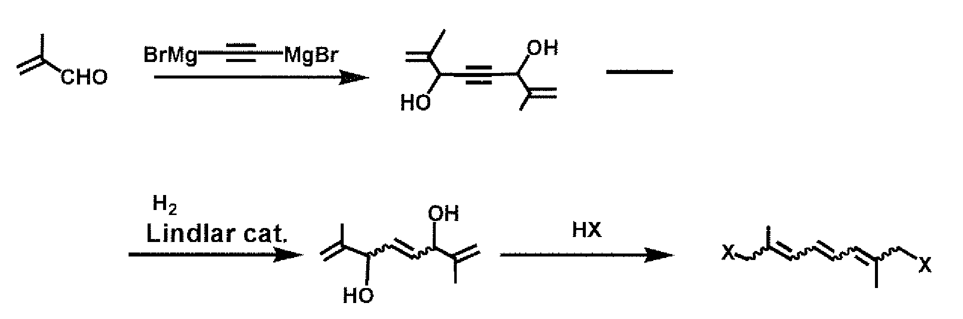 wherein M is an alkali metal or an alkaline earth metal; X is a halogen atom; and the wavy line means that the steric relation to the double bond which the wavy line is bound to is of E-form, Z-form or a mixture of E/Z.

### EXAMPLES

The present invention will be described in more detail by way of Examples, which however should not be construed as limiting the scope of the present invention in any way.

The chemical formulas of the compounds used in Examples are shown below, wherein Ts is a p-toluenesulfonyl group:

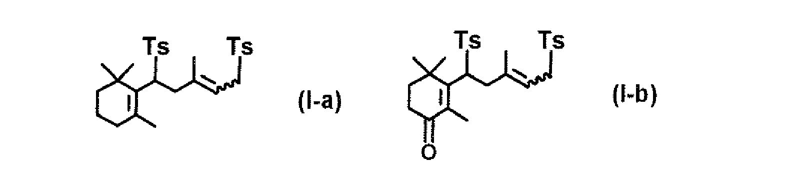

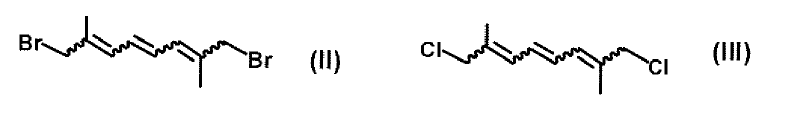

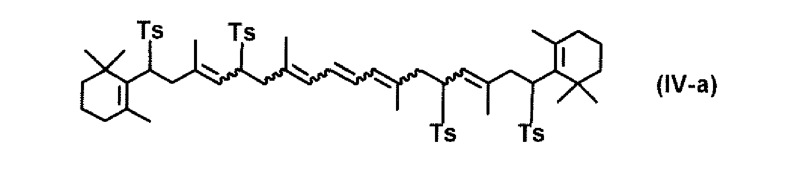

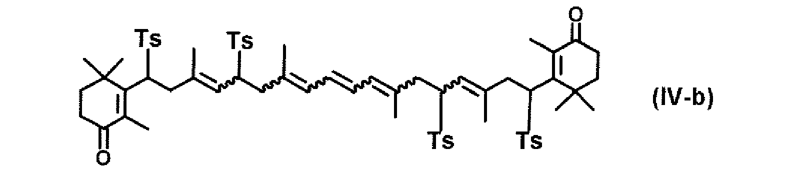

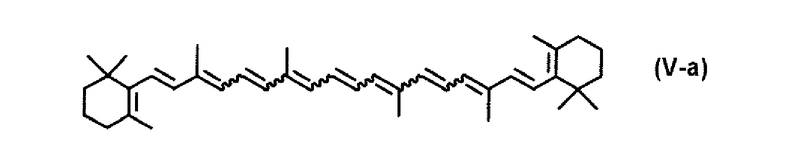

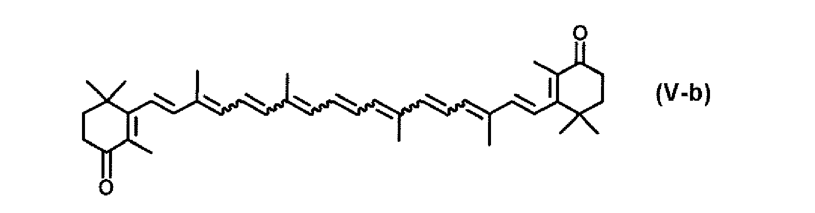

### (Example 1)

A flask was charged with 98% potassium hydroxide with a particle size of 100 µm or less (27 mg, 0.47 mmol) and dehydrated toluene (2 ml) under a nitrogen atmosphere, and was stirred at a temperature of from 20 to 30°C for 18 hours; and then, tetra-n-butylammonium bromide (6 mg, 0.02 mmol) and water (0.5 µL) were added. Next, a suspension of the compound (I-a) (98 mg, 0.19 mmol) and the compound (II) (34 mg, 0.11 mmol) in dehydrated toluene (3 ml), previously prepared, was added dropwise to the mixture at a temperature of from 25 to 30°C. After the reaction of the resulting mixture at a temperature of from 30 to 40°C for 4 hours, the dissipation of the starting compounds was confirmed. Then, the reaction mixture was quenched with water and was then neutralized with 1N hydrochloric acid and was extracted with ethyl acetate. The organic layer was dehydrated over sodium sulfate and was filtered; and the filtrate was concentrated to obtain a crude product containing the sulfone compound (IV-a). This crude product was purified by silica gel chromatography to thereby isolate the sulfone compound (IV-a) as crystals.

Analysis data:
FD-MS (m/z): 1,161
¹H-NMR (CDCl₃, δ):
0.61-2.25(m, 42H), 2.43(s, 12H), 2.48-3.07(m, 8H), 3.70-3.87(m, 4H), 4.87-4.93(m, 2H), 5.72-5.81(m, 2H), 6.12-6.17(m, 2H), 7.23-7.35(m, 8H), 7.65-7.77(m, 8H)

This compound was suggested to be the sulfone compound (IV-a) from MS and NMR.

### (Example 2)

The reaction and post-treatment were carried out in the same manners as in Example 1, except that the amount of potassium hydroxide used was 5 times larger than that used in Example 1 and the amount of tetra-n-butylammonium bromide used was 3 times larger than that used in Example 1. As a result, a mixture of the sulfone compound (IV-a) containing the compound (V-a) as a main component was obtained.

### (Example 3)

A flask was charged with 98% potassium hydroxide with a particle size of 100 µm or less (27 mg, 0.47 mmol) and dehydrated toluene (2 ml) under a nitrogen atmosphere, and was stirred at a temperature of from 20 to 30°C for 18 hours; and then, tetra-n-butylammonium bromide (6 mg, 0.02 mmol) and water (0.5 µL) were added. Next, a suspension of the compound (I-a) (98 mg, 0.19 mmol) and the compound (III) (23 mg, 0.11 mmol) in dehydrated toluene (2 ml), previously prepared, was added dropwise to the mixture at a temperature of from 25 to 30°C. After the reaction of the resulting mixture at a temperature of from 30 to 40°C for 4 hours, the dissipation of the starting compounds was confirmed. Then, the reaction mixture was quenched with water and was then neutralized with 1N hydrochloric acid and was extracted with ethyl acetate. The organic layer was dehydrated over sodium sulfate and was filtered; and the filtrate was concentrated to obtain a crude product containing the sulfone compound (IV-a). This crude product was purified by silica gel chromatography to thereby isolate the sulfone compound (IV-a) as crystals.

### (Example 4)

The reaction and post-treatment were carried out in the same manners as in Example 1, except that the amount of potassium hydroxide used was 5 times larger than that used in Example 3 and the amount of tetra-n-butylammonium bromide used was 3 times larger than that used in Example 3. As a result, a mixture of the sulfone compound (IV-a) containing the compound (V-a) as a main component was obtained.

### (Example 5)

A crude product containing the sulfone compound (IV-a) was obtained by repeating the reaction of Example 1, except that 98% potassium hydroxide with a particle size of from 100 µm to 1 mm was used.

### (Example 6)

A crude product containing the sulfone compound (IV-a) was obtained by repeating the reaction and the post-treatment of Example 2, except that 98% potassium hydroxide with a particle size of from 100 µm to 1 mm was used.

### (Example 7)

A crude product containing the sulfone compound (IV-a) was obtained by repeating the reaction and the post-treatment of Example 1, except that flake-like potassium hydroxide was used; that polyethylene glycol 600 (1% by weight, based on the weight of the potassium hydroxide) was added; and that the mixture was heated in dehydrated toluene for 2 hours under refluxing, and was then cooled to a temperature of from 20 to 30°C and was then stirred for 12 hours.

### (Example 8)

A crude product containing the sulfone compound (IV-a) was obtained by repeating the reaction and the post-treatment of Example 2, except that flake-like potassium hydroxide was used; that polyethylene glycol 600 (1% by weight, based on the weight of the potassium hydroxide) was added; and that the mixture was heated in dehydrated toluene for 2 hours under refluxing, and was then cooled to a temperature of from 20 to 30°C and was then stirred for 12 hours.

### (Example 9)

A flask was charged with 98% potassium hydroxide with a particle size of 100 µm or less (14 mg, 0.25 mmol) and toluene (containing 250 ppm of BHT) (2 ml) under a nitrogen atmosphere, and was stirred at a temperature of from 20 to 30°C for 18 hours; and then, tetra-n-butylammonium bromide (0.4 mg, 0.001 mmol) was added. Next, a suspension of the sulfone compound (IV-a) (29 mg, 0.02 mmol) in toluene (containing 250 ppm of BHT) (1 ml), previously prepared, was added dropwise to the mixture at a temperature of from 25 to 30°C. Then, methanol (0.6 µL) was added, and the mixture was reacted at a temperature of from 30 to 40°C for 10 hours. After that, the dissipation of the starting compounds was confirmed. Then, the reaction mixture was quenched with water and was then neutralized with 1N hydrochloric acid and was extracted with ethyl acetate. The organic layer was dehydrated over sodium sulfate and was filtered; and the filtrate was concentrated to obtain a crude product containing the compound (V-a). This crude product was analyzed by high-performance liquid chromatography. As a result, it was confirmed from the comparison with the reference standard that the compound (V-a) was obtained. Further, the compound purified by silica gel chromatography was subjected to MS measurement, and this compound was confirmed to be the intended product.

Analysis data:
FD-MS (m/z): 536
Conditions for analysis
Apparatus: SHIMADZU LC-10AT Model
Column: ODS A-210EC (3 mmφ X 150 mm, 5 µm)
Column temperature: 40°C
Mobile phase: solution A: water, 0.1% TFA, and
   solution B: MTA, 0.1% TFA
   B: 95% (held for 15 minutes) →
   (5 minutes) → 100% (held for 30
   minutes)
Flow rate of mobile phase: 0.5 mL/min.
Detector: UV470nm
Amount of a sample injected: 10 µL.

### (Example 10)

A flask was charged with 98% potassium hydroxide with a particle size of 100 µm or less (14 mg, 0.25 mmol) and toluene (containing 250 ppm of BHT) (2 ml) under a nitrogen atmosphere, and immediately, tetra-n-butylammonium bromide (0.4 mg, 0.001 mmol) was added. Next, a suspension of the sulfone compound (IV-a) (29 mg, 0.02 mmol) in toluene (containing 250 ppm of BHT) (1 ml), previously prepared, was added dropwise to the mixture at a temperature of from 25 to 30°C. Then, methanol (0.6 µL) was added, and the mixture was reacted at a temperature of from 30 to 40°C for 4 hours. After that, the dissipation of the starting compounds was confirmed. Then, the reaction mixture was quenched with water and was then neutralized with 1N hydrochloric acid and was then extracted with ethyl acetate. The organic layer was dehydrated over sodium sulfate and was filtered; and the filtrate was concentrated to obtain a crude product containing the compound (V-a). This crude product was analyzed by high-performance liquid chromatography. As a result, it was confirmed from the comparison with the reference standard that the compound (V-a) was obtained.

### (Example 11)

A flask was charged with 98% potassium hydroxide with a particle size of from 100 µm to 1 mm (14 mg, 0.25 mmol) and toluene (containing 250 ppm of BHT) (2 ml) under a nitrogen atmosphere, and immediately, tetra-n-butylammonium bromide (0.4 mg, 0.001 mmol) was added. Next, a suspension of the sulfone compound (IV-a) (29 mg, 0.02 mmol) in toluene (containing 250 ppm of BHT) (1 ml), previously prepared, was added dropwise to the mixture at a temperature of from 25 to 30°C. Then, methanol (0.6 µL) was added, and the mixture was reacted at a temperature of from 30 to 40°C for 4 hours. After that, the dissipation of the starting compounds was confirmed. Then, the reaction mixture was quenched with water and was then neutralized with 1N hydrochloric acid and was then extracted with ethyl acetate. The organic layer was dehydrated over sodium sulfate and was filtered; and the filtrate was concentrated to obtain a crude product containing the compound (V-a). This crude product was analyzed by high-performance liquid chromatography. As a result, it was confirmed from the comparison with the reference standard that the compound (V-a) was obtained.

### (Example 12)

A flask was charged with flake-like 98% potassium hydroxide (23 mg, 0.41 mmol) and toluene (containing 250 ppm of BHT) (2 ml) under a nitrogen atmosphere, and was stirred for one hour under refluxing and was then gradually cooled to a temperature of from 25 to 30°C. Then, tetra-n-butylammonium bromide (0.7 mg, 0.002 mmol) was added. Next, a suspension of the sulfone compound (IV-a) (50 mg, 0.04 mmol) in toluene (containing 250 ppm of BHT) (1 ml), previously prepared, was added dropwise to the mixture at a temperature of from 25 to 30°C. Then, methanol (1 µL) was added, and the mixture was reacted at a temperature of from 30 to 40°C for 10 hours. After that, the dissipation of the starting compounds was confirmed. Then, the reaction mixture was quenched with water and was then neutralized with 1N hydrochloric acid and was then extracted with ethyl acetate. The organic layer was dehydrated over sodium sulfate and was filtered; and the filtrate was concentrated to obtain a crude product containing the compound (V-a). This crude product was analyzed by high-performance liquid chromatography. As a result, it was confirmed from the comparison with the reference standard that the compound (V-a) was obtained.

### (Example 13)

A flask was charged with toluene (2 ml), the compound (I-b) (100 mg, 1.0 MR, 0.19 mmol), 95% KOH with a particle size of from 1 to 2 mm (28 mg, 2.5 MR, 0.47 mmol) and tetra-n-butylammonium bromide (3 mg, 0.05MR, 0.01 mmol) at 25°C under a nitrogen atmosphere. To the resulting slurry-like reaction mixture, a solution of the compound (III) (23 mg, 0.6 MR, 0.11 mmol) in toluene (1 ml) was added dropwise at the same temperature. Further, water (0.5 µL, 0.15 MR, 0.03 mmol) was added, and the mixture was stirred at 30°C for 6 hours. After completion of the reaction, the reaction mixture was quenched with an aqueous solution of saturated ammonium chloride and was then extracted 3 times with ethyl acetate (each 10 ml). The extract was washed with saturated brine and was dehydrated over sodium sulfate. The solvent was distilled off with an evaporator, to obtain an oily and brownish-red crude product. This crude product was subjected to quantitative determination (internal standard method) by high-performance liquid chromatography. As a result, the yield of the sulfone compound (IV-b) as a mixture of isomers was 92%, and the yield of the starting compound (I-b) was 1.5%.

Analysis data:
¹H-NMR (CDCl₃, δ):
   7.68-7.83 (4H, m), 7.58-7.72 (4H, m), 7.29-7.39 (8H, m), 6.06-6.18 (2H, m), 5.73-5.75 (2H, m), 4.83-4.91 (2H, m), 3.95-4.10 (2H, m), 3.79-3.94(2H, m), 2.69-3.10 (4H, m), 2.10-2.80 (4H, m), 2.49-2.50 (2H, m), 2.44-2.45 (12H, 4xs), 2.30-2.34 (2H, m), 2.06-2.11 (6H, 2xs), 1.76-1.90 (2H, m), 1.55-1.80 (2H, m), 1.55-1.64 (6H, 2xs), 1.20-1.27 (6H, m), 1.04-1.18 (6H, m), 0.76-1.02 (6H, m).
FD-MS: 1,189.5

### (Example 14)

A flask was charged with toluene (2 ml), the compound (I-b) (100 mg, 1.0 MR, 0.19 mmol), 95% KOH with a particle size of from 1 to 2 mm (28 mg, 2.5 MR, 0.47 mmol) and tetra-n-butylammonium bromide (3 mg, 0.05 MR, 0.01 mmol) at 25°C under a nitrogen atmosphere. To the resulting slurry-like reaction mixture, a solution of the compound (III) (23 mg, 0.6 MR, 0.11 mmol) in toluene (1 ml) was added dropwise at the same temperature. Further, water (0.5 µL, 0.15 MR, 0.03 mmol) was added, and the mixture was stirred at 30°C for 6 hours. After completion of the reaction, the reaction mixture was quenched with an aqueous solution of saturated ammonium chloride and was extracted 3 times with ethyl acetate (each 10 ml). The extract was washed with saturated brine and was dehydrated over sodium sulfate. The solvent was distilled off with an evaporator, to obtain an oily and brownish-red crude product. This crude product was subjected to quantitative determination (internal standard method) by high-performance liquid chromatography. As a result, the yield of the sulfone compound (IV-b) as a mixture of isomers was 91%, and the yield of the starting compound (1-b) was 2.1%.

### (Example 15)

A flask was charged with toluene (2 ml), the compound (I-b) (100 mg, 1.0 MR, 0.19 mmol), 95% KOH with a particle size of from 1 to 2 mm (28 mg, 2.5 MR, 0.47 mmol) and tetra-n-butylammonium bromide (3 mg, 0.05 MR, 0.01 mmol) at 25°C under a nitrogen atmosphere. To the resulting slurry-like reaction mixture, a solution of the compound (III) (23 mg, 0.6 MR, 0.11 mmol) in toluene (1 ml) was added dropwise at the same temperature. Further, water (0.5 µL, 0.15 MR, 0.03 mmol) was added, and the mixture was stirred at 30°C for 20 hours. After completion of the reaction, the reaction mixture was quenched with an aqueous solution of saturated ammonium chloride and was extracted 3 times with ethyl acetate (each 10 ml). The extract was washed with saturated brine and was dehydrated over sodium sulfate. The solvent was distilled off with an evaporator, to obtain an oily and brownish-red crude product. This crude product was subjected to quantitative determination (internal standard method) by high-performance liquid chromatography. As a result, the yield of the sulfone compound (IV-b) as a mixture of isomers was 96%, and the yield of the starting compound (I-b) was 2.8%.

### (Example 16)

The reaction and the post-treatment were carried out in the same manners as in Example 15, except that 85% KOH was used. The resulting crude product was subjected to quantitative determination (internal standard method) by high-performance liquid chromatography. As a result, the yield of the sulfone compound (IV-b) as a mixture of isomers was 94%, and the yield of the starting compound (I-b) was 4.8%.

### (Example 17)

A flask was charged in the same manner as in Example 13, except that 98% NaOH with an average particle size of 0.7 mm (20 mg) was used. The mixture was reacted at 30°C for 22 hours; and the reaction mixture was subjected to the same post-treatment as in Example 13. The resulting crude product was subjected to quantitative determination (internal standard method) by high-performance liquid chromatography. As a result, the yield of the sulfone compound (IV-b) as a mixture of isomers was 78%, and the yield of the starting compound (I-b) was 5.2%.

### (Example 18)

A flask was charged in the same manner as in Example 13, except that 98% NaOH with a particle size of from 1 to 2 mm (20 mg) was used. The mixture was reacted at 30°C for 22 hours; and the reaction mixture was subjected to the same post-treatment as in Example 13. The resulting crude product was subjected to quantitative determination (internal standard method) by high-performance liquid chromatography. As a result, the yield of the sulfone compound (IV-b) as a mixture of isomers was 73%, and the yield of the starting compound (I-b) was 2.7%.

### (Example 19)

A flask was charged in the same manner as in Example 13, except that dehydrated ethylene glycol dimethyl ether was used as the reaction solvent. The mixture was reacted at 25°C for 2 hours; and the reaction mixture was subjected to the same post-treatment as in Example 13. The resulting crude product was subjected to quantitative determination (internal standard method) by high-performance liquid chromatography. As a result, the yield of the sulfone compound (IV-b) as a mixture of isomers was 78%, and the yield of the starting compound (I-b) was 3.1%. It was also confirmed that the compound (V-b) was partially produced.

### (Example 20)

A flask was charged in the same manner as in Example 13, except that dehydrated tetrahydrofulan was used as the reaction solvent. The mixture was reacted at 25°C for 4 hours; and the reaction mixture was subjected to the same post-treatment as in Example 13. The resulting crude product was subjected to quantitative determination (internal standard method) by high-performance liquid chromatography. As a result, the yield of the sulfone compound (IV-b) as a mixture of isomers was 87%, and the yield of the starting compound (I-b) was 2%. It was also confirmed that the compound (V-b) was partially produced.

### (Example 21)

The reaction and the post-treatment were carried out in the same manners as in Example 13, except that the compound (II) (32 mg, 0.6 MR, 0.11 mmol) was used. The resulting oily and brownish-red crude product was purified by silica gel chromatography, to obtain the sulfone compound (IV-b) as yellow crystals. The yellow crystals were analyzed by high-performance liquid chromatography (area percentage). As a result, the yield of the sulfone compound (IV-b) as a mixture of isomers was 97%.

### (Example 22)

A flask was charged with toluene (3 ml), 95% KOH with a particle size of from 100 µm to 1 mm (50 mg, 10.0 MR, 0.84 mmol) and tetra-n-butylammonium bromide (1 mg, 0.05 MR, 0.004 mmol) at 25°C under a nitrogen atmosphere. To the resulting slurry-like reaction mixture, a solution of the sulfone compound (IV-b) (100 mg, 1.0 MR, 0.08 mmol) in toluene (2 ml) was added at 25°C. Methanol (1.3 µL, 0.5 MR, 0.04 mmol) was added, and the mixture was stirred at 30°C for 20 hours. The reaction was observed by way of HPLC and TLC. At a point of time when the starting compound and the reaction intermediate had been substantially dissipated, the reaction mixture was quenched with an aqueous solution of 1N hydrochloric acid and was then extracted 3 times with ethyl acetate (each 10 ml). The extract was washed with saturated brine, and the organic layer was dehydrated over sodium sulfate. The solvent was distilled off to obtain a solid. This solid was analyzed by HPLC (using a PDA detector). As a result, it was confirmed from the comparison with the reference standard sample that the compound (V-b, as a mixture of isomers) was obtained at a yield of 93% (area percentage).

### INDUSTRIAL APPLICABILITY

According to the present invention, sulfone compounds useful as intermediates for carotenoids, and carotenoids can be produced by commercially advantageous processes.

## Claims

1. A process for producing a sulfone compound of the following formula (3), **characterized in that** an allyl sulfone compound of the formula (1) and an allyl halide compound of the formula (2) are reacted in an organic solvent in the presence of an alkali metal hydroxide and a phase-transfer catalyst: 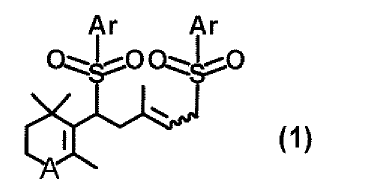 wherein A is CH₂ or C=O; Ar is an aryl group optionally having 1 to 3 substitutents; and the wavy line means that the steric relation to the double bond which the wavy line is bound to is of E-form, Z-form or a mixture of E/Z; 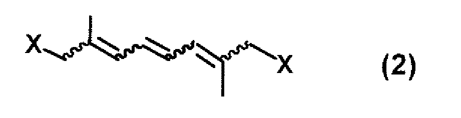 wherein X is a halogen atom; and the wavy line means the same as defined above; and 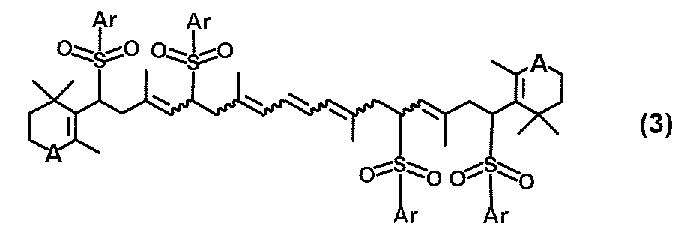 wherein A, Ar and the wavy line mean the same as defined above.

2. A process for producing a carotenoid, **characterized in that** an allyl sulfone compound of the formula (1) and an allyl halide compound of the formula (2) are reacted in an organic solvent in the presence of an alkali metal hydroxide and a phase-transfer catalyst; and an alcohol is added to the resulting reaction mixture: 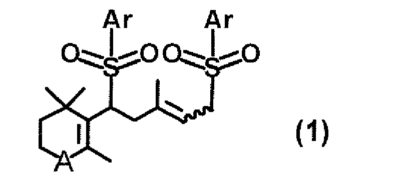 wherein A is CH₂ or C=O; Ar is an aryl group optionally having 1 to 3 substitutents; and the wavy line means that the steric relation to the double bond which the wavy line is bound to is of E-form, Z-form or a mixture of E/Z; and 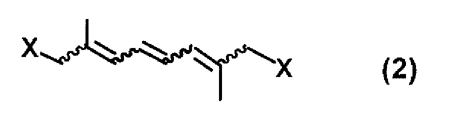 wherein X is a halogen atom; and the wavy line means the same as defined above.

3. The process of Claim 1 or 2, wherein in the compound of the formula (1) Ar is a 4-methylphenyl group.

4. The process of any one of Claims 1 to 3, wherein in the allyl halide compound of the formula (2) X is a bromine atom or a chlorine atom.

5. The process of Claim 4, wherein X is a chlorine atom.

6. The process of any one of Claims 1 to 5, wherein the alkali metal hydroxide is potassium hydroxide or sodium hydroxide, having a purity of 85% or higher.

7. The process of any one of Claims 1 to 6, wherein the particle size of the alkali metal hydroxide is 3 mm or less.

8. The process of Claim 7, wherein the particle size of the alkali metal hydroxide is 100 µm or less.

9. The process of any one of Claims 1 to 8, wherein the amount of the alkali metal hydroxide to be used is from 1 to 30 times larger, in terms of mole, than that of the allyl sulfone compound of the formula (1).

10. The process of any one of Claims 1 to 9, wherein the phase-transfer catalyst is a quaternary ammonium salt.

11. The process of any one of Claims 1 to 10, wherein the amount of the phase-transfer catalyst to be used is from 0.01 to 0.5 times larger, in terms of mole, than that of the ally sulfone compound of the formula (1).

12. The process of any one of Claims 1 to 11, wherein water is added in an amount from 0.05 to 0.5 times larger, in terms of mole, than that of the ally sulfone compound of the formula (1).

13. The process of any one of Claims 1 to 12, wherein the organic solvent is an aromatic hydrocarbon- or ether-based solvent.

14. A process for producing a carotenoid, **characterized in that** a sulfone compound of the formula (3) is reacted in an organic solvent in the presence of an alkali metal hydroxide and a phase-transfer catalyst: 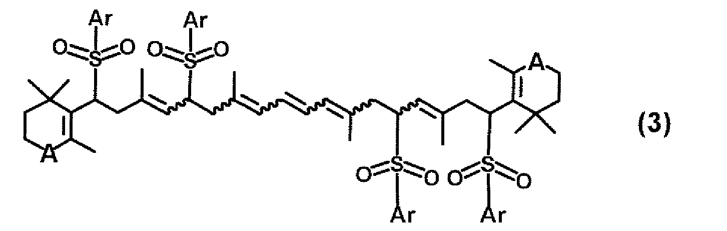 wherein A is CH₂ or C=O; Ar is an aryl group optionally having 1 to 3 substitutents; and the wavy line means that the steric relation to the double bond which the wavy line is bound to is of E-form, Z-form or a mixture of E/Z.

15. The process of Claim 14, wherein in the compound of the formula (3) Ar is a 4-methylphenyl group.

16. The process of Claim 14 or 15, wherein the alkali metal hydroxide is potassium hydroxide or sodium hydroxide, having a purity of 85% or higher.

17. The process of any one of Claims 14 to 16, wherein the particle size of the alkali metal hydroxide is 3 mm or less.

18. The process of Claim 17, wherein the particle size of the alkali metal hydroxide is 100 µm or less.

19. The process of any one of Claims 14 to 18, wherein the phase-transfer catalyst is a quaternary ammonium salt.

20. The process of any one of Claims 14 to 19, wherein a C₁-C₅ lower alcohol is added.

21. The process of any one of Claims 14 to 20, wherein the organic solvent is an aromatic hydrocarbon- or ether-based solvent.

22. A sulfone compound of the formula (3): 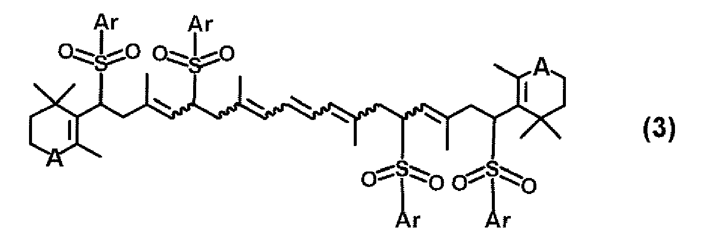 wherein A is CH₂ or C=O; Ar is an aryl group optionally having 1 to 3 substitutents; and the wavy line means that the steric relation to the double bond which the wavy line is bound to is of E-form, Z-form or a mixture of E/Z.

23. The sulfone compound of Claim 22, wherein Ar is a 4-methylphenyl group.
